# EUROPEAN PATENT SPECIFICATION

(11) **EP 1 429 657 B1**
(45) Date of publication and mention of the grant of the patent: **23.03.2005**
(21) Application number: 02773108.2
(22) Date of filing: 27.09.2002
(51) Int. Cl.: A61B 5/22

(54) **AN APPARATUS FOR EVALUATING MANUAL DEXTERITY**
VORRICHTUNG ZUR BEWERTUNG MANUELLER FINGERFERTIGKEIT
APPAREIL PERMETTANT D'EVALUER LA DEXTERITE MANUELLE

(30) Priority: 28.09.2001 SE 0103238
(43) Date of publication of application: 23.06.2004
(73) Proprietor: Johansson, Roland Sixten, 907 38 Umea (SE)
(72) Inventor: Johansson, Roland Sixten, 907 38 Umea (SE)
(74) Representative: Onn, Thorsten
(86) International application number: PCT/SE2002/001767
(87) International publication number: WO 2003/026507

(56) References cited:
- WO-A1-87/02567
- US-A- 5 174 154
- FOTHERGILL D.M. ET AL.: 'Protocol for the omnidirectional assessment of manual strength' CLIN. BIOMECH. vol. 8, 1993, pages 27 - 134, XP000362806
- PINDER A.D.J. T AL.: 'System for the triaxial measurement of manual force exertions' CLIN. BIOCHEM. vol. 8, 1993, pages 120 - 126, XP000362805

## Description

The present invention relates to an apparatus for evaluating manual dexterity in humans and in particular to an apparatus for evaluating the manual dexterity of humans with an impaired nervous system and/or musculoskeletal system.

Manual dexterity of humans can provide useful information in relation to their nervous system and/or musculoskeletal system. This information is required for a variety of reasons ranging from pre-operative, post-operative and ongoing assessment of functional abnormalities in patients for adapting and optimising training protocols, to prognosis and diagnosis of patients. Additionally, evaluation of manual dexterity can provide valuable information for insurance companies for whom accurate assessment of the recovery of accident victims is vital to prevent against fraudulent claims.

A variety of apparatuses have been manufactured and a number of methods have been devised in order to assist medics and other interested parties with the complex technique of assessing and evaluating manual dexterity in humans. US Patent No. 5,174,154 discloses an isometric force-measuring pinch meter having first and second opposed force-bearing surfaces. An incompressible, nonfluid material is positioned between the first and second surfaces and is capable of building up pressure in response to the application of force to the force-bearing surfaces. A pressure transducer is responsive to the pressure stored in the material for producing an output signal representative of the force. The apparatus is for use with either hand for measuring pinch strength and for ease of application of the fingers and thumb about the apparatus. It also removes any inaccuracies induced by misalignment of the thumb and fingers on the device itself.

US Patent No. 4,674,330 discloses an apparatus for measurement of both the grip strength of a person's hand and the pinch strength of two digits of the hand. The apparatus comprises two parallel grip handles extending at right angles from a third member. The handles are rigidly fixed to the third member and have two pairs of strain gauges to measure the force applied to the handles irrespective of where that force is applied. The ends of the handles terminate in flat portions to be used for measurement of pinch strength. Again, the apparatus is for use with either hand for measuring both grip strength and pinch strength. US Patent No. 4,878,384 discloses a device for evaluating and measuring human sensory perception by measuring muscle force applied against a finger button and displaying a maximum muscle force. US Patent No. 5,449,002 discloses an apparatus for recording the mechanical pressure applied by a patient recovering from injury or suffering from a disability during routine physical functions such as gripping by the hand. The apparatus utilises a technique known as biofeedback to help patients relearn functions or prevent complications that impede functions. German Patent No. DE 4,209,193 discloses an apparatus known as an Ergo graph for measuring the grip of a patients hand in addition to a method for measuring the personality changes in a patient as a result of brain damage suffered by the patient.

A substantial proportion of nervous system or musculoskeletal impairments are unilateral and therefore a person having such an impairment may have one hand in perfect working order. In order to fully asses the extent of the impairment of the nervous system and/or the musculoskeletal system it is essential to have a set of control data from a fully functional unimpaired hand to compare the data received from a damaged hand with.

All of the apparatuses and methods disclosed in the patent specifications discussed above are for operation by a single hand and designed to record forces applied by a subject in one direction only. However, manual dexterity and object manipulation is a three dimensional activity which often involves bimanual engagement. Any attempt to evaluate manual dexterity should take these facts into consideration.

Clearly there is a need for an improved apparatus and method for quantitatively assessing manual dexterity of a human in order to evaluate the level of impairment of the nervous system and the musculoskeletal system as a result of an accident or as a result of a mental or physical disability.

Accordingly, there is provided an apparatus for evaluating manual dexterity and object manipulation in humans comprising a means for connecting two hands wherein each hand is capable of transmitting a force onto the other hand via the connecting means and the apparatus comprises a means for simultaneously measuring and recording omnidirectional forces applied by each hand. Torques applied by the subject can also be recorded.

In one aspect of the invention, one person holds the connecting means between both hands. The apparatus allows simultaneous measurement of the capacities of the left and the right hand. Thus, with unilateral impairments the contra lateral (unimpaired) hand will immediately serve as a reference.

In a further aspect of the invention, the connecting means is held by two people, with each person holding the apparatus by one hand. Beneficially, if one of the people, an experimenter holds the connecting means at one end and the second person, a subject holds the connecting means at the other end, both the experimenters hand and the subjects hand can be evaluated independently.

Preferably, the connecting means is provided by a hand-held unit. This removes the influence of all variables except gravity and forces applied by subjects holding the hand held unit.

Preferably, the apparatus further comprises a control unit in communication with the measuring and recording means for recording and analysing forces received by the measuring and recording means. On-line communication between the control unit and the measuring and recording means increases the functionality of the apparatus.

Preferably, the control unit is in communication with the measuring and recording means telemetrically. This allows the apparatus to take advantage of digital telemetric techniques that are available as silicon chips.

In another embodiment, the control unit and the measuring and recording means are physically connected by electrical cable.

Ideally, the control unit and the measuring and recording means are physically integrated into one unit.

Ideally, the control unit is provided by a computer and a variety of peripheral devices such as speakers and a display screen.

Preferably, the computer is configured by a customised control program. The control program is an essential feature of the invention providing the apparatus with on-line functionality.

Ideally, the hand-held unit comprises a pair of handles attachable about either end of a joining member wherein the joining member transmits forces between said handles.

In one embodiment, the handles are fixed to the joining member.

In another embodiment, the handles are rotatable about the longitudinal axis of the joining member.

Ideally, each handle has a pair of grip surfaces for receiving any of the digits of a person's hand. The primary purpose of the apparatus and the associated analysis procedure is the efficient quantitative assessment of the functionality of digits of the subjects' hands in terms of force coordination for grasp stability under different grasp loading conditions and the ability to symmetrically apply fingertip forces about opposite grip surfaces of the handles during precision manipulation. Thus, reduction in control of the digits as a result of both impairments in the nervous system and impairments in the musculoskeletal apparatus will be revealed.

In a preferred embodiment, the grip surfaces are provided by longitudinally extending hemi-cylindrical ridges co-axial with the longitudinal axis of the joining member when the handles are mounted on the joining member. This simplifies assessment of the subject's ability to choose advantageous contact points on the grip surfaces by focusing on their longitudinal dimension. Healthy subjects avoid applying asymmetric forces by opposing digits because it creates unnecessary internal forces within the handles (i.e., uneconomical tangential forces that, in turn, have to be matched by higher grip forces).

Preferably, the distance between the grip surfaces is in the range of 10 to 40 millimetres. This allows the apparatus to be used across a wide range of subjects including children and adults having a variety of hand sizes. One area of potential use for the apparatus is thus for following the ontogenetic development of sensorimotor functions. Today, the routine clinical methods that are used to identify children with sensorimotor impairments are very basic and provide little quantitative evaluation.

Preferably, the handles of the apparatus are equipped with transducers to measure omnidirectional forces generated by the subject at each of the grip surfaces. The transducers permit measurements of coordination between grip forces and tangential forces at each of the two grip surfaces. For each hand, the transducers measure the opposing grip forces applied in directions normal to the length axis of the hand held unit and the load force applied tangential to the grip surfaces, i.e. push/pull forces applied along the length axis of the hand held unit and twist forces (torques) applied and around this axis. The force tangential to the gripped surfaces at each digit is measured together with torques around the centre of grip force pressure. The total tangential load can be computed based on these measurements.

Ideally, the transducers measure internal forces due to asymmetric applications of the gripping forces produced by opposing digits at each grip surface along with measurements of the points of force pressure centres.

In a preferred embodiment, the handles are attachable about either end of the joining member. This reduces the complexity of the overall apparatus and reduces the number of parts to be designed and manufactured.

Preferably, the handles are interchangeable with handles having alternative geometries.

Preferably, handles having parallel, tapered and curved surfaces are used. Obtaining grip stability on different surface geometries requires changes in balance between the grip force and tangential force applied about the grip surfaces. Thus, sensory information about surface geometry is used to adjust the force balance. Grip stability implies that subjects apply grip forces large enough to avoid accidental slips, while at the same time excessive grip forces are avoided, which naturally may harm the apparatus, injure the hand or cause unnecessary muscle fatigue.

Concerning changes in surface geometry, a powerful variation is implemented by tapered grip surfaces. If the grip surfaces of the handles are tapered towards each other at the end of the handles distal from the joining member healthy subjects apply higher grip forces when they pull on the handle for maintenance of grip stability in comparison to parallel grip surfaces. With grip surfaces of the handles tapered towards one another at the end of the handles proximate to the joining member, stronger forces are required when applying push forces.

In a preferred embodiment, the geometry of each handle is adjustable by mechanical, electrical or other non-manual means.

In one embodiment, a shield is placed between the subject and the handles. This prevents the subject using their vision to adjust the balance required between grip-load force coordination, in particular when working with changes in the geometry of the grip surfaces. It is necessary to avoid visual cues when testing the capacity of the sensibility of the digits to mediate adaptation of the balance between the grip and load forces to variations in the surface geometry.

Preferably, the joining member comprises two sections and a coupling mounted there between.

In a preferred embodiment, the two sections are inter-engaged to allow compression and extension of the joining member along their longitudinal axis in response to push/pull forces applied by a subject and the coupling applies a predetermined biasing force opposing the compression and extension of the joining member.

In a preferred embodiment, the coupling is in communication with the computer and the biasing force applied by the coupling can be momentarily removed in response to a signal from the computer and subsequently reapplied. The coupling between a left and a right section of the joining member is temporarily broken and subsequently latched. A load rebound occurs when the coupling is latched. This transient load impact triggers automatic gripping force increases based on signals from sensors in the hand in a healthy subject. Results taken from this test can be used to obtain useful information in relation to the nervous system and the musculoskeletal system.

Preferably, the computer generates the signal at random times during the test. This will increase the likelihood of obtaining genuine responses from subjects and therefore improve the accuracy of the test.

Alternatively, the signal is generated by the computer in response to a predetermined time having elapsed from commencement of the test, in response to a certain force applied to the coupling by the subject or in response to a certain distance travelled by the two sections of the joining member as a result of a force applied by the subject.

Additionally, the computer generates signals providing information for subjects taking the test.

Preferably, the information includes a demonstration and instructions for the subject taking the test.

Ideally, the signals may be audible, visible or tangible. This facilitates people with visual and/or audio deficiencies.

Preferably, the signals include various target forces for subjects to aim for. This provides an element of control to the evaluation process and allows an experimenter to set limits for a subject to aim for.

In one embodiment, the signal is output as a visual display on a screen. This allows for efficient and interactive communication with the subject in terms of providing instructions for the desired actions.

In one embodiment, the screen is a freestanding unit located in front of the subject.

In a preferred embodiment, the screen is positioned on the joining member between the two handles. This helps to focus the subject's attention entirely on the hand-held unit. In another embodiment, the signal is output as an audible signal via speakers. This allows people with impaired vision or blindness to successfully operate the apparatus.

Preferably, the computer comprises a means for receiving and storing input forces measured by the transducers.

Ideally, the computer comprises a means for comparing the forces received with a set of control values which are input by the experimenter.

Preferably, the computer generates a signal informing the subject that they have successfully completed the current section of the test. This signal is only output after the comparison of current input forces with control values has been carried out and the input forces are acceptable for further processing. This removes the possibility of unsuccessful tests due to unusable data being collected thereby saving time and money.

In a particularly preferred embodiment, the computer instantaneously outputs a signal containing the values of current input tangential forces being received at the grip surfaces and being measured by the transducers to the subject either audibly, visually or tangibly thereby updating the subject on the progress being made.

In a still further aspect of the invention, the visual display includes a pre-test demonstration using graphical symbols or characters showing the subject what they are required to do.

The invention will now be described with reference to the accompanying drawings and test procedure, which show and outline, by way of example only, one embodiment of an apparatus for evaluating manual dexterity in humans in accordance with the invention. In the drawings:
Figure 1 is a schematic drawing showing an elevation view of the apparatus;
Figure 2 is a schematic end view of one handle of the apparatus;
Figure 3 is a schematic elevation view of a hand held unit including a display screen;
Figure 4 is a schematic drawing of a hand held unit showing a coupling;
Figure 5 is a schematic drawing of a hand held unit showing a shield;
Figure 6 is a schematic elevation view of a hand held unit having one combination of handles;
Figure 7 is a schematic elevation view of a hand held unit having another combination of handles;
Figure 8 is a schematic elevation view of a hand held unit having another combination of handles;
Figure 9 is a schematic elevation view of a hand held unit having a still further combination of handles;
Figure 10 is a representational graph showing the results of a test conducted using parallel and tapered grip surfaces; and
Figure 11 is a schematic drawing showing one possible visual display to be output on a display screen;

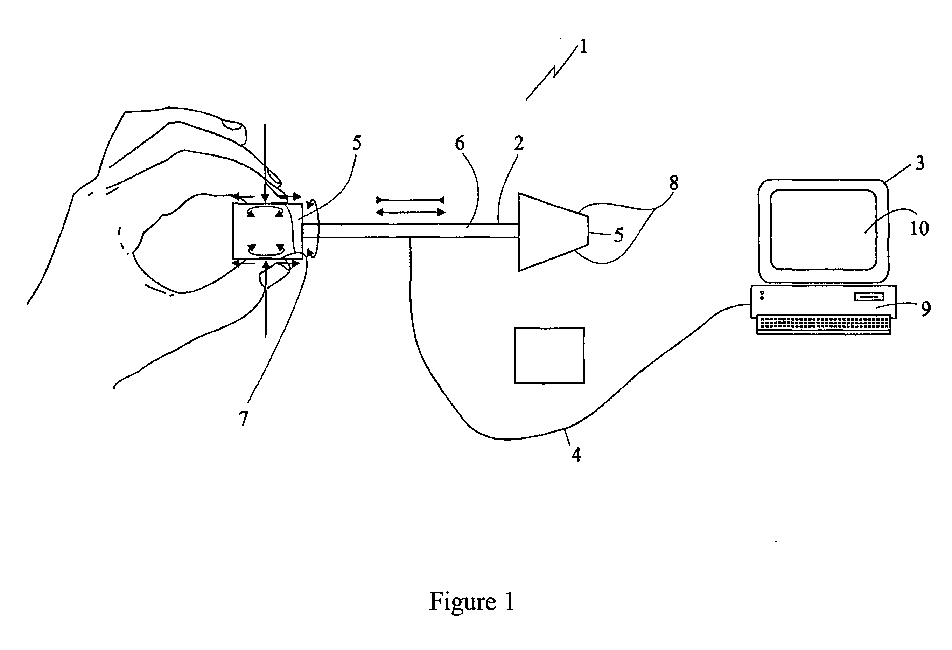

Referring to the drawings and initially to Figure 1 there is shown an apparatus for evaluating manual dexterity and object manipulation in humans indicated generally by the reference numeral 1.The apparatus 1 comprises a hand held unit 2 and a control unit 3. The control unit 3 is connected to the hand held unit 2 by electrical cable 4. The hand held unit 2 comprises a pair of handles 5 and a joining member 6. The handle 5 on the left hand side of the unit 2 has parallel grip surfaces 7 and the handle 5 on the right hand side of the hand held unit 2 has tapered grip surfaces 8. Transducers (not shown) are fitted to the grip surfaces 7 and 8 and additionally on or about the connection between the handles 5 and the joining member 6. Straight and curved arrows indicate examples of directions of forces and torques measured by fitted transducers. The control unit 3 comprises a computer 9 having a monitor 10 and speakers (not shown).

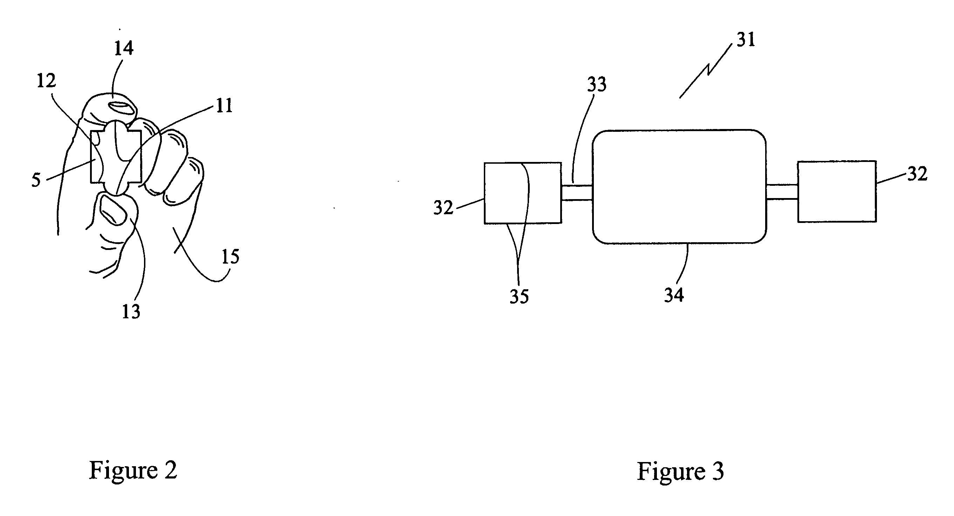

Referring now to Figure 2, there is shown a handle 5 of the hand held unit 2. The grip surfaces 12 of the handle 5 are provided by longitudinally extending hemi-cylindrical ridges 11. The ridges 11 are co-axial with the longitudinal axis of the joining member 6 when the handle 5 is mounted on said member 6. The handle 5 is pinched on grip surfaces 12 between a thumb 13 and an index finger 14 of a subject's hand 15. The distance between grip surfaces 12 is typically between 10 mm and 40 mm. In Figure 3, there is shown a hand held unit 31 having a pair of handles 32, a joining member 33 and a display screen 34. The handles 32 have parallel grip surfaces 35 and the display screen 34 is mounted on the joining member 33 of the hand held unit 31.

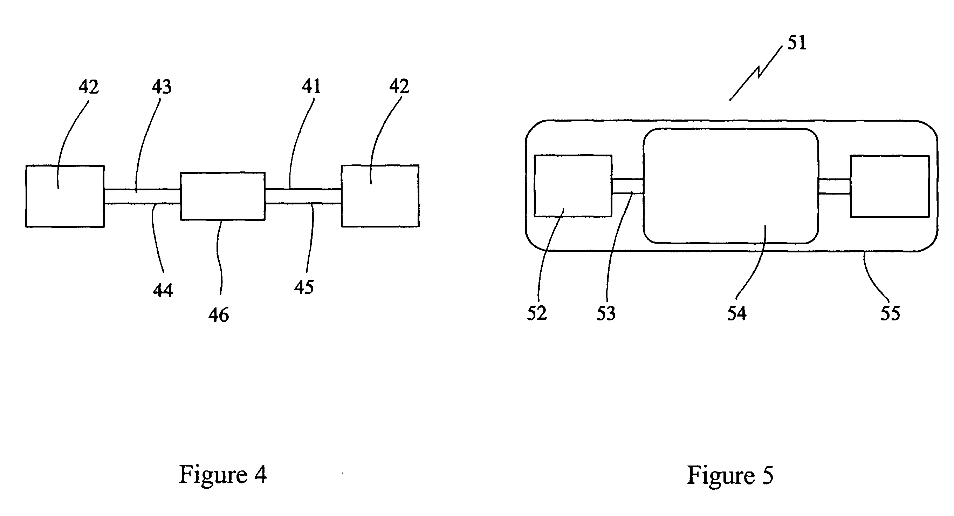

Referring now to Figure 4, there is shown a hand held unit 41 having a pair of handles 42 and a joining member 43. The joining member 43 has two sections 44 and 45 and a coupling 46 mounted between the two sections 44 and 45. The two sections 44, 45 are inter-engaged to allow compression and extension of the hand held unit 41 about the longitudinal axis of the joining member 43. The coupling 46 is designed to oppose the extension and compression of the hand held unit 41 and is also designed to allow a momentary removal of the opposing force and subsequent latching. Figure 5 shows a further embodiment of the invention wherein a hand held unit 51 has a pair of handles 52, a joining member 53, a display screen 54 and a shield 55. In this example, the shield 55 is mounted between the hand held unit 51 and the display screen 54 in order to prevent a subject from seeing the shape of the handles 52 on the hand held unit 51.

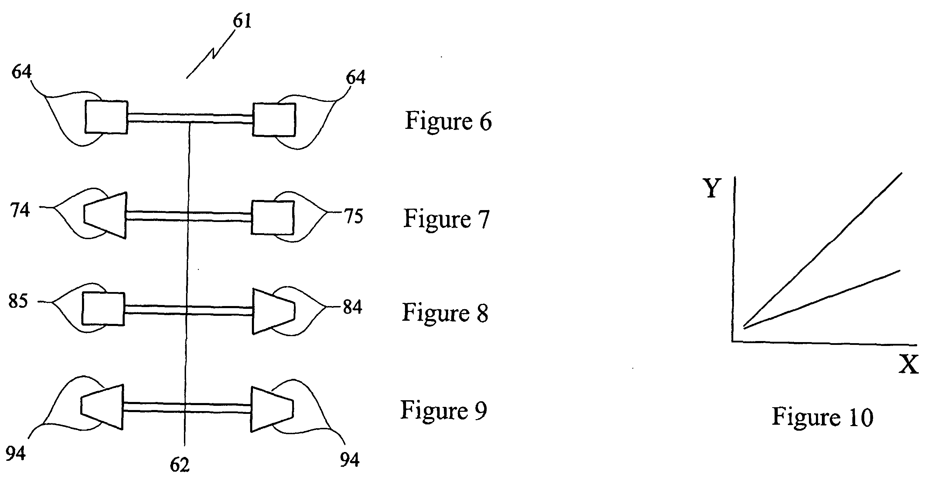

Figures 6 to 9 each show a hand held unit indicated generally by the reference numeral 61. The units 61 each have a joining member 62 and a pair of handles with various geometries. Figure 6 shows a hand held unit 61 with both the left handle and the right handle having parallel grip surfaces 64. Figure 7 shows a hand held unit 61 with the left handle having grip surfaces 74 tapered at 30° to the horizontal and the right handle having parallel grip surfaces 75. Figure 8 shows a hand held unit 61 with the right handle having grip surfaces 84 tapered at 30° to the horizontal and the left handle having parallel grip surfaces 85. Figure 9 shows a hand held unit 61 with both the left handle and the right handle having grip surfaces 94 tapered at an angle of 30° to the horizontal.

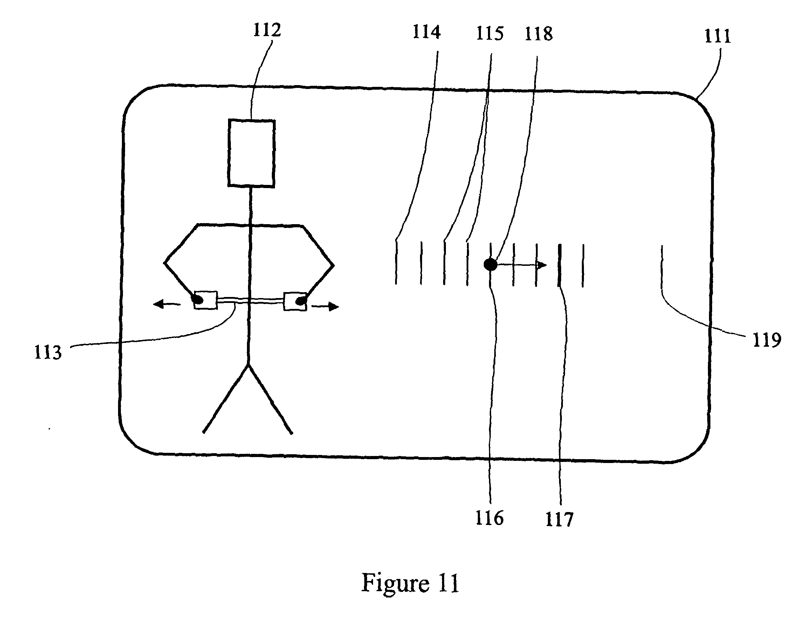

Referring to the drawings and now to Figure 10 there is shown a representative graph of pull force on the x-axis versus grip force on the y-axis. Line 101 represents a handle with parallel grip surfaces and line 102 represents a handle with tapered grip surfaces. Finally, Figure 11 shows one possible visual display generated by the computer 9 when configured by the control program and output via a display screen 111. An image of an electronic stick-man 112 holding a hand held unit 113 demonstrates what a subject is required to do. A visual display of a scale 114 is marked with vertical lines 115 and displays force. This scale 114 shows a subject the zero force marker 116 and the target force marker 117 by highlighting the relevant line 115 along the scale 114. The dot 118 shows the current force. A target for maximum pull force 119 is located at the right end of the scale.

In use, a subject or an examiner starts a test by striking any key on a keyboard of the computer 9. This initiates the demonstration on the display screen 34, 54, 111 or initiates an audible demonstration. The audible demonstration includes a list of verbal instructions output via speakers connected to the computer 9 and is useful for a subject with impaired vision or blindness. When a visual demonstration is used, a pull or push prompt is displayed on the computer screen 34, 35, 111 and in response the stick man 112 stretches or compresses the electronic hand held unit 113 in order to show a subject the correct action to take in response to a specific prompt. Within the same display screen 34, 54, 111, the scale 114 shows the effect of the stick man 112 stretching or compressing the electronic units 113 by simultaneously moving the current force marker 118 between the zero force marker 116 and the target force marker 117.

Once a subject is satisfied with the demonstration, they can proceed with the test by striking any key on the keyboard of the computer 9. The hand held unit 2, 31, 41, 51, 61 is held by a subject by the grip surfaces 7, 8, 12, 35, 64, 74, 75, 84, 85 and 94 between digits of their hands. The subject then follows a list of prompts displayed on the display screen 34, 54, 111 or a list of verbal instructions output via speakers which are connected to the computer 9. The display also includes the scale 114 which shows the effect of the subject stretching or compressing the hand held unit 2, 31, 41, 51, 61 by simultaneously moving the current force marker 118 between the zero force marker 116 and the target force marker 117. The visual display may also include a prompt for the subject to maintain the target force for a predetermined period of time. Once the target force has been attained, the computer 9 monitors the quality of the data received from the transducers (not shown) comparing it with control data. The computer 9 outputs a signal to the display screen 34, 54, 111 informing the subject to release the applied force if the data received is acceptable. Alternatively, these prompts can be output via the speakers. The subject follows the audible or visual instructions until completion of the test. In order to prevent the subject using their vision to alter grip-load force co-ordination a shield 55 is placed between the hand held unit 2, 31, 41, 51, 61 and the subject for certain parts of the test.

The hand held unit 2, 31, 41, 51, 61 is grasped bimanually and manipulated according to a pre-defined (standardized) protocol. An automated test protocol combined with automated data analysis allows efficient assessment of the subject's manual status based on the data obtained from the force/torque transducers of units 2, 31, 41, 51 or 61.

There now follows a detailed description of an example of a test protocol and data analysis.
1. The subject familiarizes themselves with the apparatus 1 by doing a few ramp-and-hold pull forces and push forces to certain target forces (e.g. +4 Newtons (N), -4N). Before the action, the task is demonstrated on the display screen 34, 54, 111 by the icon stickman 112 performing the task. The subject's task is to copy the demonstration.
2. The subject performs a series of ramp-and-hold pull forces of different magnitudes according to the predetermined protocol (e.g., 1, 4, 2, 8 Newtons (N)) three times followed by a series of push forces (e.g. 4, 4, 4 N). These tests are repeated for the different geometric configurations of the handles, shown in the drawings. Quality control of the data occurs on-line and is dependent upon the subject maintaining the target push/pull force (within a predefined tolerance) for two seconds. In response to successful completion of the task by the subject, the target marker 118 on the display screen 34, 54, 111 is turned off. An audible signal indicating that this section of the test has been successfully completed could also be generated.
   Analysis: The computer 9 configured by the control program automatically reads and stores the measured variables during a 2 second epoch while the subject maintains the target force. The coordination between grip force and the load at each hand (and at the level of the individual's digits) is automatically analyzed. The subject's capacity to scale the grip force with the load force and to adjust the balance between the grip force and load with changes in geometry of grip surfaces 7, 8, 12, 35, 64, 74, 75, 84, 85 and 94 is assessed for each hand.
3. The subject makes sinusoidal pull forces at different frequencies. The amplitude (e.g. 4N with approximately +/-20% tolerance) is guided by the display screen 34, 54, 111. A metronome sound guides the pace. The frequency range to be explored is 0.5 - 3 Hz. Again, quality control of the data occurs on-line. Before changing to the next frequency, a certain number of cycles with an accepted amplitude and frequency are collected. (The grip surfaces 7, 12, 35, 64, 75, 85 are parallel on all handles.)
   Analysis: The phase between grip force and the load is analysed; normally there is no phase lag, which indicates that the grip force predicts adequately the self-generated load forces (for grip stability). The depth of grip force modulation is assessed as a function of frequency; normally there is a steep decrease in the modulation with increased frequency, starting at about 1.5 - 2 Hz.
4. The subject makes sinusoidal pull-push forces at different frequencies (0.5 - 1.5 Hz) and at constant push-pull amplitudes (e.g. -4N (push) to +4N (pull)). (The grip surfaces 7, 12, 35, 64, 75, 85 are parallel on all handles.) In this task, the absolute value of the tangential load increases and decreases with twice the frequency of the sinusoidal load. Normal subjects respond to this "frequency doubling" by generating two grip force increases per load cycle i.e. the grip force predicts the actual load although it does not match the frequency of the load cycles.
   Analysis: The phase between grip force and the absolute value of the load and the depth of grip force modulation is analyzed (cf. above). This analysis assesses the subject's ability to generalize the directional consequences of self-generated fingertip loads in terms of grip force requirements (and thus grip force predictions).
5. The subject produces their maximum bimanual pull force. Normally, a subject's right hand is stronger than their left- yet subjects never allow the left hand to slip off the unit 2, 31, 41, 51, 61 during bimanual operations because the knowledge of their musculoskeletal system is incorporated as a control constraint in the neural networks of the brain. To assess asymmetries of force generating capacity by the two hands, the maximum grip force is assessed for each hand separately.
6. Finally, by imposing the transient load impact at a few unpredictable points during the test protocol numbered 2 (see above) the reflex status of the reactive grip force control of the two hands is assessed. Normally, with two healthy hands, the load impact triggers automatic grip force increases at both hands with similar amplitudes and onset latencies.

The results of the tests provided the following information.

Impairment of sensory functions of the digits is reflected in:
1. A weakened or lost capacity of the impaired hand to adjust the balance between the grip forces and the load forces in response to changes in geometry of the grip surfaces 7, 8, 12, 35, 64, 74, 75, 84, 85 and 94.
2. With sensory impairment of one hand, the accompanying healthy hand also controls the grip-load force coordination of the numb hand. Likewise, the healthy hand uses strategies that reflect constraints imposed by the impaired hand during bimanual actions.
3. The impaired hand generates increased internal forces upon the hand held unit 2, 31, 41, 51 or 61 due to asymmetric grip force applications at the opposing grip surfaces 7, 8, 12, 35, 64, 74, 75, 84, 85 and 94. This causes unnecessary/uneconomical load of the grasp.

Impairment of predictive control of grip forces is reflected in:
1. A temporal mismatch between the grip and load force changes and is revealed during the test with sinusoidal load changes.
2. A reduced modulation of grip forces with load force changes compared to healthy conditions.

Impairment of reactive control of grip forces is reflected in:
One or several of the following items revealed impaired reactive grip force control: (1) No response to the transient load impact. (2) A prolonged onset latency of the reactive grip force increases. (3) Smaller amplitudes than normal of the reactive grip force increases. With unilateral impairments, asymmetries between the hands in these respects are important.

Impairment of the brain's proprioceptive knowledge:
With impaired proprioceptive knowledge concerning one diseased hand the healthy contralateral hand shows incompetence in controlling its actions based on constraints imposed by the diseased hand during bimanual tasks. Impaired proprioceptive knowledge is assessed in the present protocol by an inability of the healthy hand to limit the application of pull force to the hand-held unit as to match the capacity of the companion diseased hand. This is critically revealed during the test of maximum voluntary bimanual pull along the hand-held unit 2, 31, 41, 51, 61. With proprioceptive knowledge the hand-held unit 2, 31, 41, 51, 61 does not slip at the diseased hand, whereas with loss of proprioceptive knowledge the unit 2, 31, 41, 51, 61 slips from the subjects grip.

## Claims

1. An apparatus (1) for evaluating manual dexterity and object manipulation in humans comprising a means for connecting two hands wherein each hand is capable of transmitting a force onto the other hand via the connecting means and the apparatus (1) comprises a means for measuring and recording omnidirectional forces applied by each hand.

2. An apparatus (1) as claimed in claim 1, wherein the connecting means is adapted to be held by one person between both hands.

3. An apparatus (1) as claimed in claim 1, wherein the connecting means is adapted to be held by two people, with each person holding the apparatus (1) by one hand.

4. An apparatus (1) as claimed in any preceding claims 1 to 3, wherein the connecting means is provided by a hand-held unit (2, 31, 41, 51, 61).

5. An apparatus (1) as claimed in any preceding claim, wherein the apparatus (1) further comprises a control unit (3) in communication with the measuring and recording means for recording and analyzing forces received by the measuring and recording means.

6. An apparatus (1) as claimed in claim 5, wherein the control unit (3) is provided by a computer (9) and a variety of peripheral devices such as speakers and a display screen (10).

7. An apparatus (1) as claimed in claim 6, wherein the computer (9) is configured by a customised control program.

8. An apparatus (1) as claimed in any of claims 4 to 7, wherein the hand-held unit (2, 31, 41, 51, 61) comprises a pair of handles (5) attachable about either end of a joining member (6) and in use the joining member (6) transmits forces between said handles (5).

9. An apparatus (1) as claimed in claim 8, wherein the handles (5) are fixed to the joining member (6).

10. An apparatus (1) as claimed in claim 8, wherein the handles (5) are rotatable about the longitudinal axis of the joining member (6).

11. An apparatus (1) as claimed in any of claims 8 to 10, wherein transducers are fitted between the handles (5) and the joining member (6) in order to measure the torque applied by the subject to each handle (5) of the hand-held unit (2, 31, 41, 51, 61).

12. An apparatus (1) as claimed in any of claims 8 to 11, wherein each handle (5) has a pair of grip surfaces (7, 8, 12, 35, 64, 74, 75, 84, 85, 94) for receiving any of the digits of a person's hand.

13. An apparatus (1) as claimed in any of claims 8 to 12, wherein the grip surfaces (7, 8, 12, 35, 64, 74, 75, 84, 85, 94) are provided by longitudinally extending hemi-cylindrical ridges (11) co-axial with the longitudinal axis of the joining member (6) when the handles (5) are mounted on the joining member.

14. An apparatus (1) as claimed in claim 12 and claim 13, wherein the distance between the grip surfaces (7, 8, 12, 35 ,64, 74, 75, 84, 85, 94) is in the range of 10 to 40 millimetres.

15. An apparatus (1) as claimed in any of claims 8 to 14, wherein the handles (5) of the apparatus (1) are equipped with transducers to measure omnidirectional forces generated by the subject at each of the grip surfaces (7, 8, 12, 35, 64, 74, 75, 84, 85, 94).

16. An apparatus (1) as claimed in claim 11, wherein the transducers measure internal forces due to asymmetric applications of the gripping forces produced by opposing digits at each grip surface (7, 8, 12, 35, 64, 74, 75, 84, 85, 94) along with measurements of the points of force pressure centres.

17. An apparatus (1) as claimed in any of claims 8 to 16, wherein the handles (5) are attachable about either end of the joining member (6).

18. An apparatus (1) as claimed in any of claims 8 to 17, wherein the handles (5) are interchangeable with handles having alternative geometries.

19. An apparatus (1) as claimed in any of claims 8 to 18, wherein the handles (5) have parallel, tapered or curved surfaces.

20. An apparatus (1) as claimed in any of claims 8 to 19, wherein the geometry of each handle (5) is adjustable by mechanical, electrical or other non-manual means.

21. An apparatus (1) as claimed in any of claims 8 to 20, wherein a shield (55) is placed between the subject and the handles (5).

22. An apparatus (1) as claimed in any of claim 1 and claims 8 to 21, wherein the joining member (6) comprise two sections (44, 45) and a coupling (46) mounted between the sections.

23. An apparatus (1) as claimed in claim 22, wherein the two sections are inter-engaged to allow compression and extension of the members along their longitudinal axis in response to push/pull forces applied by a subject and the coupling applies a predetermined biasing force opposing the compression and extension of said members.

24. An apparatus (1) as claimed in claims 22 and 23, wherein the coupling (46) is in communication with the computer (9) and the biasing force applied by the coupling can be momentarily removed in response to a signal from the computer and subsequently reapplied.

25. An apparatus (1) as claimed in claim 24, wherein the computer (9) generates the signal at random times during the test.

26. An apparatus (1) as claimed in 24 and 25, wherein the signal is generated by the computer (9) in response to a predetermined time having elapsed from commencement of the test, in response to a certain force applied to the coupling by the subject or in response to a certain distance travelled by the two sections of either member as a result of a force applied by the subject.

27. An apparatus (1) as claimed in any of claims 6 to 26, wherein the computer (9) generates signals providing information for subjects taking the test.

28. An apparatus (1) as claimed in claim 27, wherein the information includes a demonstration and instructions for the subject taking the test.

29. An apparatus (1) as claimed in any of claims 27 and 28, wherein the signals maybe audible, visible or tangible.

30. An apparatus (1) as claimed in any of claims 27 to 29, wherein the signals include various target forces for subjects to aim for.

31. An apparatus (1) as claimed in any of claims 27 to 30, wherein the signals are output as a visual display on a screen (34, 54, 111).

32. An apparatus (1) as claimed in any of claims 27 to 30, wherein the signal is output as an audible signal via speakers.

33. An apparatus (1) as claimed in claim 31, wherein, the screen (34, 54, 111) is a freestanding unit located in front of the person.

34. An apparatus (1) as claimed in claim 31, wherein the screen is mounted on the joining member between the two handles.

35. An apparatus (1) as claimed in any of claims 6 to 34, wherein the computer (9) comprises a means for receiving and storing input forces measured by the transducers.

36. An apparatus (1) as claimed in any of claims 6 to 35, wherein the computer (9) comprises a means for comparing the forces received with a set of control values which are input by a the experimenter.

37. An apparatus (1) as claimed in any of claims 6 to 36, wherein the computer generates a signal informing the person that they have successfully completed the current section of the test.

38. An apparatus (1) as claimed in any of claims 31 to 37, wherein the visual display includes a pre-test demonstration using graphical symbols or characters showing the subject what they are required to do.

## Patentansprüche

1. Ein Apparat (1) für das Bewerten der manuellen Geschicklichkeit und Handhabung von Gegenständen beim Menschen, der ein Mittel zum Verbinden von zwei Händen beinhaltet, wobei jede Hand fähig ist, über das verbindende Mittel eine Kraft auf die andere Hand zu übertragen und der Apparat (1) ein Mittel für das Messen und Aufzeichnen von durch jede Hand ausgeübten omnidirektionalen Kräften beinhaltet.

2. Ein Apparat (1) gemäss Anspruch 1, wobei das verbindende Mittel darauf angepasst ist, von einer Person zwischen beiden Händen gehalten zu werden.

3. Ein Apparat (1) gemäss Anspruch 1, wobei das verbindende Element darauf angepasst ist, von zwei Personen gehalten zu werden, wobei jede Person den Apparat (1) mit einer Hand hält.

4. Ein Apparat (1) gemäss einem der vorhergehenden Ansprüche 1 bis 3, wobei das verbindende Element durch ein Handgerät (2, 31, 41, 51, 61) zur Verfügung gestellt wird.

5. Ein Apparat (1) gemäss einem der vorhergehenden Ansprüche, wobei der Apparat (1) des weiteren eine Steuereinheit (3) beinhaltet, die mit den Mess- und Aufzeichnungsmitteln in Kommunikation steht um von den Mess- und Aufzeichnungsmitteln empfangene Kräfte aufzuzeichnen und zu analysieren.

6. Ein Apparat (1) gemäss Anspruch 5, wobei die Steuereinheit (3) durch einen Computer (9) und eine Anzahl von Peripheriegeräten wie Lautsprecher und einen Anzeigeschirm (10) zur Verfügung gestellt wird.

7. Ein Apparat (1) gemäss Anspruch 6, wobei der Computer (9) durch ein kundenspezifisches Steuerprogramm konfiguriert ist.

8. Ein Apparat (1) gemäss einem der Ansprüche 4 bis 7, wobei das Handgerät (2, 31, 41, 51, 61) ein Paar Handgriffe beinhaltet (5), die um jedes Ende eines Verbindungsglieds (6) befestigbar sind und im Gebrauch das Verbindungsglied (6) Kräfte zwischen besagten Handgriffen (5) übertragt.

9. Ein Apparat (1) gemäss Anspruch 8, wobei die Handgriffe (5) am Verbindungsglied (6) befestigt sind.

10. Ein Apparat (1) gemäss Anspruch 8, wobei die Handgriffe (5) um die Längsachse des Verbindungsglieds (6) drehbar sind.

11. Ein Apparat (1) gemäss einem der Ansprüche 8 bis 10, wobei Wandler zwischen den Handgriffen (5) und dem Verbindungsglied (6) befestigt sind, um das Drehmoment zu messen, das von der Testperson auf jeden Handgriff (5) des Handgeräts (2, 31, 41, 51, 61) ausgeübt wird.

12. Ein Apparat (1) gemäss einem der Ansprüche 8 bis 11, wobei jeder Handgriff (5) ein Paar Griffflächen (7, 8, 12, 35, 64, 74, 75, 84, 85, 94) für die Aufnahme beliebiger Finger der Hand einer Person hat.

13. Ein Apparat (1) gemäss einem der Ansprüche 8 bis 12, wobei die Griffflächen (7, 8, 12, 35, 64, 74, 75, 84, 85, 94) durch longitudinal verlaufende hemizylindrische Wulste (11) zur Verfügung gestellt werden, die mit der Längsachse des Verbindungsglieds (6) koaxial sind, wenn die Handgriffe (5) am Verbindungsglied angebracht sind.

14. Ein Apparat (1) gemäss Anspruch 12 und Anspruch 13, wobei der Abstand zwischen den Griffflächen (7, 8, 12, 35, 64, 74, 75, 84, 85, 94) im Bereich von 10 bis 40 Millimetern liegt.

15. Ein Apparat (1) gemäss Ansprüche 8 bis 14, wobei die Handgriffe (5) des Apparates (1) mit Wandlern ausgerüstet sind, um die omnidirektionalen Kräfte zu messen, die durch die Testperson an jeder der Griffflächen (7, 8, 12, 35, 64, 74, 75, 84, 85, 94) erzeugt werden.

16. Ein Apparat (1) gemäss Anspruch 11, wobei die Wandler die internen Kräfte aufgrund der asymmetrischen Ausübung der Griffkräfte, die durch entgegengesetzte Finger an jeder Griffoberfläche (7, 8, 12, 35, 64, 74, 75, 84, 85, 94) produziert werden messen, zusammen mit Messungen der Positionen der Kraftdruckmitten.

17. Ein Apparat (1) gemäss einem der Ansprüche 8 bis 16, wobei die Handgriffe (5) um jedes Ende des Verbindungselement (6) befestigbar sind.

18. Ein Apparat (1) gemäss einem der Ansprüche 8 bis 17, wobei die Handgriffe (5) gegen Handgriffe mit alternativen Geometrien austauschbar sind.

19. Ein Apparat (1) gemäss einem der Ansprüche 8 bis 18, wobei die Handgriffe (5) parallele, sich zuspitzende oder gebogene Oberflächen haben.

20. Ein Apparat (1) gemäss einem der Ansprüche 8 bis 19, wobei die Geometrie jedes Handgriffs (5) mechanisch, elektrisch oder andere nicht-manuelle Mittel anpassbar ist.

21. Ein Apparat (1) gemäss einem der Ansprüche 8 bis 20, wobei ein Sichtschutz (55) zwischen der Testperson und den Handgriffen (5) platziert ist.

22. Ein Apparat (1) gemäss einem der Ansprüche 1 und 8 bis 21, wobei das Verbindungselement (6) zwei Abschnitte (44, 45) beinhaltet sowie eine Kupplung (46), die zwischen den Abschnitten angebracht ist.

23. Ein Apparat (1) gemäss Anspruch 22, wobei die beiden Abschnitte ineinandergreifen, um als Reaktion auf durch eine Testperson aufgewendete Druck-/Zugkräfte die Kompression und Verlängerung der Glieder entlang ihrer Längsachse zu erlauben und die Kupplung eine vorbestimmte Vorspannkraft aufbringt, die der Kompression und der Verlängerung der besagten Glieder entgegenwirkt.

24. Ein Apparat (1) gemäss einem der Ansprüche 22 oder 23, wobei die Kupplung (46) in Kommunikation zu dem Computer (9) steht, und die Vorspannkraft, die durch die Kupplung aufgebracht wird, als Reaktion auf ein Signal vom Computer kurzzeitig entfernt und nachher wieder aufgebracht werden kann.

25. Ein Apparat (1) gemäss Anspruch 24, wobei der Computer (9) das Signal zu zufälligen Zeitpunkten während des Tests erzeugt.

26. Ein Apparat (1) gemäss einem der Ansprüchen 24 oder 25, wobei das Signal durch den Computer (9) als Reaktion auf den Ablauf einer vorbestimmten Zeit erzeugt wird, die seit dem Anfang des Tests verstrichen ist, als Reaktion auf eine bestimmte Kraft, die durch die Testperson auf der Kupplung ausgeübt wurde, oder als Reaktion auf eine bestimmte Strecke, die die zwei Abschnitte jedes Glieds als Ergebnis der von der Testperson aufgewendeten Kraft zurückgelegt haben.

27. Ein Apparat (1) gemäss einem der Ansprüche 6 bis 26, wobei der Computer (9) Signale erzeugt, die Informationen für die Personen bereitstellen, die getestet werden.

28. Ein Apparat (1) gemäss Anspruch 27, wobei die Information eine Demonstration und Anweisungen für die Testperson beinhaltet.

29. Ein Apparat (1) gemäss einem der Ansprüche 27 oder 28, wobei die Signale hörbar, sichtbar oder fühlbar sein können.

30. Ein Apparat (1) gemäss einem der Ansprüche 27 bis 29, wobei die Signale verschiedene Zielkräfte miteinschließen, die die Testpersonen anstreben sollen.

31. Ein Apparat (1) gemäss einem der Ansprüche 27 bis 30, wobei die Signale als Sichtanzeige auf einem Schirm (34, 54, 111) ausgegeben werden.

32. Ein Apparat (1) gemäss einem der Ansprüche 27 bis 30, wobei das Signal als hörbares Signal über Lautsprecher ausgegeben wird.

33. Ein Apparat (1) gemäss Anspruch 31, wobei der Bildschirm (34, 54, 111) eine freistehende Einheit ist, die sich vor der Person befindet.

34. Ein Apparat (1) gemäss Anspruch 31, wobei der Bildschirm am Verbindungselement zwischen den beiden Handgriffen angebracht ist.

35. Ein Apparat (1) gemäss einem der Ansprüche 6 bis 34, wobei der Computer (9) Mittel für das Empfangen und Speichern der Eingangskräfte enthält, die durch die Wandler gemessen werden.

36. Ein Apparat (1) gemäss einem der Ansprüche 6 bis 35, wobei der Computer (9) Mittel enthält, um die erfassten Kräfte mit einem Satz von Kontrollwerten zu vergleichen, die durch den Experimentator eingegeben werden.

37. Ein Apparat (1) gemäss einem der Ansprüchen 6 bis 36, wobei der Computer ein Signal erzeugt, welches die Person informiert, daß sie den gegenwärtigen Abschnitt des Tests erfolgreich durchgeführt haben.

38. Ein Apparat (1) gemäss einem der Ansprüche 31 bis 37, wobei die Sichtanzeige eine dem Test vorausgehende Demonstration unter Verwendung graphischer Symbole oder Buchstaben enthält, die der Testperson zeigen, was von ihnen zu tun verlangt wird.

## Revendications

1. Un appareil (1) pour évaluer la dextérité manuelle et la manipulation des objets chez l'homme comportant un moyen pour connecter deux mains où chaque main est capable de transmettre une force sur l'autre main par l'intermédiaire du moyen de connexion et l'appareil (1) comporte des moyens pour mesurer et enregistrer des forces omnidirectionnelles appliquées par chaque main.

2. Un appareil (1) selon la revendication I, où le moyen de connexion est adapté d'être tenu par une personne entre les deux mains.

3. Un appareil (1) selon la revendication I, où le moyen de connexion est adapté d'être tenu par deux personnes, avec chaque personne tenant l'appareil (1) par une main.

4. Un appareil (1) selon l'une quelconque des revendications précédentes 1 à 3, où le moyen de connexion est mis à disposition par une unité à main (2, 31, 41, 51, 61).

5. Un appareil (1) selon l'une quelconque des revendications précédentes, où l'appareil (1) comporte en plus une unité de contrôle (3) etant en communication avec les moyens de mesure et d'enregistrement pour l'enregistrement et l'analyse des forces reçues par les moyens de mesure et d'enregistrement.

6. Un appareil (1) selon la revendication 5, où l'unité de contrôle (3) est fournie par un ordinateur (9) et une variété de périphériques tels que des haut-parleurs et un écran de visualisation (10).

7. Un appareil (1) selon la revendication 6, où l'ordinateur (9) est configuré par un programme de contrôle particularisé.

8. Un appareil (1) selon l'une des revendications 4 à 7, où l'unité à main (2, 31, 41, 51, 61) comporte une paire de manettes (5) attachables à chaque extrémité d'un membre de jonction (6) et en service le membre de jonction (6) transmet des forces entre lesdites manettes (5).

9. Un appareil (1) selon la revendication 8, où les manettes (5) sont fixées au membre de jonction (6).

10. Un appareil (1) selon la revendication 8, où les manettes (5) sont rotatives autour de l'axe longitudinal du membre de jonction (6).

11. Un appareil (1) selon l'une des revendications 8 à 10, où des capteurs sont agencés entre les manettes (5) et le membre de jonction (6) afin de mesurer le couple appliqué par le sujet à chaque manette (5) de l'unité à main (2, 31, 41, 51, 61).

12. Un appareil (1) selon l'une des revendications 8 à 11, où chaque manette (5) a une paire de surfaces de préhension (7, 8, 12, 35, 64, 74, 75, 84, 85, 94) pour recevoir des doigts quelconques de la main d'une personne.

13. Un appareil (1) selon l'une des revendications 8 à 12, où les surfaces de préhension (7, 8, 12, 35, 64, 74, 75, 84, 85, 94) sont fournies par des arêtes hemi-cylindriques (11) s'étendant longitudinalement, qui sont coaxiales avec l'axe longitudinal du membre de jonction (6) lorsque les manettes (5) sont montées sur le membre de jonction.

14. Un appareil (1) selon la revendication 12 et la revendication 13, où la distance entre les surfaces de préhension (7, 8, 12, 35, 64, 74, 75, 84, 85, 94) est dans la gamme de 10 à 40 millimètres.

15. Un appareil (1) selon l'une des revendications 8 à 14, où les manettes (5) de l'appareil (1) sont équipées des capteurs pour mesurer les forces omnidirectionnelles produites par le sujet sur chacune des surfaces de préhension (7, 8, 12, 35, 64, 74, 75, 84, 85, 94).

16. Un appareil (1) selon la revendication 11, où les capteurs mesurent des forces internes dues à des applications asymétriques des forces de préhension produites par des doigts s'opposant sur chaque surface de préhension (7, 8, 12, 35, 64, 74, 75, 84, 85, 94), ensemble avec des mesures des points de centres de pression de force.

17. Un appareil (1) selon l'une des revendications 8 à 16, où les manettes (5) sont attachables à l'une ou l'autre extrémité du membre de jonction (6).

18. Un appareil (1) selon l'une des revendications 8 à 17, où les manettes (5) sont interchangeables avec des manettes ayant des géometries alternatives.

19. Un appareil (1) selon l'une des revendications 8 à 18, où les manettes (5) ont des surfaces parallèles, effilés ou courbés.

20. Un appareil (1) selon l'une des revendications 8 à 19, où la géométrie de chaque manette (5) est réglable par moyens mécaniques, électriques ou d'autres moyens non manuels.

21. Un appareil (1) selon l'une des revendications 8 à 20, où un bouclier (55) est placé entre le sujet et les manettes (5).

22. Un appareil (1) selon l'une des revendications 1 et 8 à 21, où le membre de jonction (6) comporte deux sections (44, 45) et un accouplement (46) monté entre les sections.

23. Un appareil (1) selon la revendication 22, où les deux sections sont engrenées pour permettre la compression et la prolongation des membres le long de leur axe longitudinal en réponse aux forces de pression/traction appliquées par un sujet et l'accouplement applique une force de précontrainte prédéterminée s'opposant à la compression et à la prolongation desdits membres.

24. Un appareil (1) selon les revendications 22 ou 23, où l'accouplement (46) est en communication avec l'ordinateur (9) et la force de précontrainte appliquée par l'accouplement peut être momentanément enlevé en réponse à un signal de l'ordinateur et être plus tard réappliquée.

25. Un appareil (1) selon la revendication 24, où l'ordinateur (9) produit le signal à des moments incidentaire pendant le test.

26. Un appareil (1) selon les revendications 24 ou 25, où le signal est produit par l'ordinateur (9) en réponse à un temps prédéterminé s'étant écoulé depuis le commencement de l'essai, en réponse à une certaine force appliquée à l'accouplement par le sujet ou en réponse à une certaine distance accomplie par les deux sections de l'un ou l'autre membre en raison d'une force appliquée par le sujet.

27. Un appareil (1) selon l'une des revendications 6 à 26, où l'ordinateur (9) produit des signaux fournissant des informations pour des sujets prenant le test.

28. Un appareil (1) selon la revendication 27, où l'information inclut une démonstration et des instructions pour le sujet prenant le test.

29. Un appareil (1) selon l'une des revendications 27 ou 28, où les signaux peuvent être audibles, visibles ou tangibles.

30. Un appareil (1) selon l'une des revendications 27 à 29, où les signaux incluent de diverses forces de cible à viser par les sujets.

31. Un appareil (1) selon l'une des revendications 27 à 30, où les signaux sont produits comme visualisation sur un écran (34, 54, 111).

32. Un appareil (1) selon l'une des revendications 27 à 30, où le signal est émis comme signal audible par l'intermédiaire de haut-parleurs.

33. Un appareil (1) selon la revendication 31, où l'écran (34, 54, 111) est une unité indépendante placé devant la personne.

34. Un appareil (1) selon la revendication 31, où l'écran est monté sur le membre de jonction entre les deux manettes.

35. Un appareil (1) selon l'une des revendications 6 à 34, où l'ordinateur (9) comporte des moyens pour recevoir et enregistrer des forces d'entrée mesurées par les capteurs.

36. Un appareil (1) selon l'une des revendications 6 à 35, où l'ordinateur (9) comporte des moyens pour comparer les forces reçues à un ensemble de valeurs de contrôle qui sont entrées par l'expérimentateur.

37. Un appareil (1) selon l'une des revendications 6 à 36, où l'ordinateur produit un signal informant la personne qu'elle a avec succès accompli la section courante de l'essai.

38. Un appareil (1) selon l'une des revendications 31 à 37, où la visualisation inclut une démonstration prétest utilisant des symboles ou des caractères graphiques montrant aux sujets ce qu'ils doivent faire.
